# EUROPÄISCHE PATENTSCHRIFT

(11) **EP 3 941 548 B1**
(45) Veröffentlichungstag und Bekanntmachung des Hinweises auf die Patenterteilung: **11.12.2024**
(21) Anmeldenummer: 20713550.0
(22) Anmeldetag: 19.03.2020
(51) Int. Cl.: A61M 1/16, A61M 1/36

(54) **EXTRAKORPORALE BLUTBEHANDLUNGSMASCHINE MIT POKA YOKE FÜR DRUCKAUFNEHMER**
EXTRACORPOREAL BLOOD TREATMENT MACHINE COMPRISING A POKA-YOKE FOR A PRESSURE SENSOR
MACHINE DE TRAITEMENT DE SANG EXTRACORPORELLE COMPORTANT UN SYSTÈME ANTI-ERREUR DESTINÉ À UN CAPTEUR DE PRESSION

(30) Priorität: 19.03.2019 DE 102019107031
(43) Veröffentlichungstag der Anmeldung: 26.01.2022
(73) Patentinhaber: B. Braun Avitum AG, 34212 Melsungen (DE)
(72) Erfinder: BRÖGGER, Sebastian, 34593 Knüllwald (DE); OCHSE, Michael Dr., 34212 Melsungen (DE); RITTER, Kai-Uwe, 34212 Melsungen (DE); THOMAS, Thorsten, 34613 Schwalmstadt (DE)
(74) Vertreter: Winter, Brandl - Partnerschaft mbB
(86) Internationale Anmeldenummer: PCT/EP2020/057642
(87) Internationale Veröffentlichungsnummer: WO 2020/188040

(56) Entgegenhaltungen:
- EP-A1- 3 061 473
- JP-A- S61 113 461
- US-A- 5 895 571
- US-A1- 2015 367 062
- US-A1- 2019 001 041

## Beschreibung

### Technisches Gebiet

Die vorliegende Offenbarung betrifft ein System aus einer extrakorporalen Blutbehandlungsmaschine, insbesondere Dialysemaschine, mit einer Blutbehandlungsvorrichtung, insbesondere einer Filterkartusche (Dialysator), einer Fördervorrichtung zum Fördern von Blut durch die Blutbehandlungsvorrichtung und einer Anschlussmaske, und aus einem an die extrakorporale Blutbehandlungsmaschine angepassten Schlauchset. Die Anschlussmaske ist dazu ausgebildet, das angepasste Schlauchset in einer vorbestimmten Anordnung an der Blutbehandlungsmaschine austauschbar aufzunehmen. Das Schlauchset weist zumindest eine Blutzuführleitung zum Zuführen von Blut zur Blutbehandlungseinheit, eine Blutrückführleitung zum Rückführen von behandeltem Blut von der Behandlungseinheit sowie zumindest zwei Übertragungsleitungen auf. Die Übertragungsleitungen gehen jeweils an einer Abzweigung von der Blutzuführleitung oder der Blutrückführleitung ab und sind mit an der Blutbehandlungsmaschine angeordneten Druckaufnehmeranschlüssen verbindbar, um einen Druck von den Abzweigungen zum entsprechenden Druckaufnehmeranschluss zu übertragen.

### Stand der Technik

Die Blutschlauchleitungen an extrakorporalen Behandlungsmaschinen, wie bspw. Dialysemaschinen, müssen drucküberwacht werden. Das dient dazu, die dort herrschenden Drücke in einem physiologisch kompatiblen Bereichen einstellen und im Falle kritischer Druckzustände schnell handeln zu können. Zu diesem Zweck sind Abzweige an den Blutschlauchleitungen vorgesehen, die mittels dünner Schläuche (Drucküberwachungsleitungen bzw. Druckübertragungsleitungen) an maschinenseitige Druckaufnehmeranschlüsse kontaktiert sind. Eine solche Drucküberwachungsleitung kann bspw. über ein T-Stück mit dem Schlauch verbunden sein. Zwischen einer in der Drucküberwachungs-/Druckmessleitung stehenden Flüssigkeitssäule und dem Druckaufnehmer befindet sich dann ein Luftpolster. Dieses Luftpolster verändert sich bei Druckänderung im Schlauch (dehnt sich aus oder wird komprimiert, was wiederum zu einer Auslenkung des Druckaufnehmers führt.

Typischerweise weist z.B. eine Dialysemaschine einen Druckaufnehmeranschluss zum Überwachen des arteriellen Unterdrucks (PA) und einen Druckaufnehmeranschluss zum Überwachen des venösen Drucks (PV) auf.

Die Druckaufnehmeranschlüsse sind gängiger Weise nahe beieinander und in einem oberen Bereich der Maschine (oberhalb interner Fluidsysteme und in der Nähe der restlichen Elektronikkomponenten) angeordnet. Die Schlauchlängen der Drucküberwachungsleitungen weisen in bekannten Systemen bzw. Schlauchsets in der Regel eine deutliche Überlänge auf. Dies hat zur Folge, dass die Drucküberwachungsleitungen bei der Montage oder dem Austausch eines Schlauchsets an einen falschen Druckaufnehmeranschluss der Maschine angeschlossen werden können. Zum Vermeiden eines solchen Vertauschens sind die Druckaufnehmeranschlüsse bislang beschriftet.

Ein solches Vertauschen wird im besten Fall während eines Selbsttests von der Blutbehandlungsmaschine erkannt, welche daraufhin einen Alarm auslöst. Dies hat zur Folge, dass Bedienpersonal kommen und den Fehler beheben muss, indem die Anschlüsse manuell korrekt konnektiert werden.

Systeme aus einer extrakorporalen Blutbehandlungsmaschine und aus einem Schlauchset sind beispielsweise aus der EP 3 061 473 A1, der JP S61 113461 A und der US 5 895 571 A bekannt. Die US 2015/0367062 A1 offenbart ein Schlauchset.

### Kurzbeschreibung der Offenbarung

Aufgabe der vorliegenden Erfindung ist es deshalb eine extrakorporale Blutbehandlungsmaschine mit einfacherer Bedienung bzw. einfacherer Montage eines austauschbaren Schlauchsets bereitzustellen.

Diese Aufgabe wird durch die Merkmale des unabhängigen Anspruchs 1 gelöst. Vorteilhafte Weiterbildungen sind Gegenstand der Unteransprüche.

Offenbarungsgemäß ist eine extrakorporale Blutbehandlungsmaschine mit einer Blutbehandlungsvorrichtung zum Behandeln des Blutes eines Patienten, einer Fördervorrichtung, zum Fördern von Blut durch die Blutbehandlungsvorrichtung über einen extrakorporalen Blutkreislauf und einer Anschlussmaske (Schnittstelle) für den extrakoporalen Blutkreislauf vorgesehen. Die Anschlussmaske ist dazu ausgebildet, ein angepasstes Schlauchset in einer vorbestimmten Anordnung an der Blutbehandlungsmaschine austauschbar aufzunehmen. Das Schlauchset weist zumindest eine Blutzuführleitung (arterieller Schlauchabschnitt) zum Zuführen von Blut zur Blutbehandlungseinheit, eine Blutrückführleitung (venöser Schlauchabschnitt) zum Rückführen von behandeltem Blut von der Behandlungseinheit auf. Somit gibt das Schlauchset den extrakorporalen Blutkreislauf vor. Das Schlauchset weist zudem zumindest zwei Drucküberwachungsleitungen auf. Die Drucküberwachungsleitungen gehen jeweils an einer Abzweigung von der Blutzuführleitung oder der Blutrückführleitung ab. Zudem sind sie mit maschinenseitig an der Anschlussmaske angeordneten Druckaufnehmeranschlüssen verbindbar und dazu angepasst, einen Druck aus einem Abschnitt des Schlauchsets über die Abzweigungen zum entsprechenden Druckaufnehmeranschluss zu übertragen. Die Druckaufnehmeranschlüsse sind derart (ausreichend weit) voneinander beabstandet und in ihrer Positionierung an der extrakorporalen Blutbehandlungsmaschine auf das Schlauchset abgestimmt, dass, wenn das Schlauchset in der durch die Anschlussmaske vorgegebenen, vorbestimmten Anordnung montiert ist, jede der Drucküberwachungsleitungen aufgrund ihrer begrenzten Länge und der vorbestimmten Anordnung der zugehörigen Abzweigung an der Blutbehandlungsmaschine ausschließlich mit je einem der Druckaufnehmeranschlüsse verbindbar ist. In anderen Worten ist in einer Blutbehandlungsmaschine der Abstand zwischen zwei Druckaufnehmeranschlüssen (Konnektoren) an der Maschinenfront so ausgewählt, dass sich ein bestimmter Effekt ergibt, wenn das Schlauchset in der vorbestimmten Anordnung an der Anschlussmaske montiert ist. Der Abstand ist so gewählt, dass der Radius, der sich von der zugehörigen Abzweigung aus gesehen zwischen Drucküberwachungsleitungs-Anfang und Drucküberwachungsleitungs-Ende ergibt, nur das Anschließen an den gewünschten Druckaufnehmeranschluss zulässt.

Auf diese Weise lässt sich ein Poka-Yoke-System für die Druckaufnehmeranschlüsse implementieren, das die Bedienung der Maschine vereinfacht und Montagezeiten für das Anbringen des Schlauchsets reduziert. Die oben beschriebene Anschlussmaske wird beispielsweise durch die Fördervorrichtung und die Blutbehandlungsvorrichtung ausgebildet. Diese definieren die Lage und Ausrichtung von Schlauchset-Abschnitten bzw. Abschnitten des extrakorporalen Blutkreislaufs. Die Anschlussmaske kann darüber hinaus noch weitere Komponenten aufweisen, beispielsweise Sensoren, Okklusionsklemmen, Halter für Luftfallen und dergleichen.

Bevorzugt kann die Blutbehandlungsmaschine eine Dialysemaschine und die Blutbehandlungsvorrichtung eine Dialysator-Filterkartusche, Hämofiltrations-Kartusche oder dergleichen sein.

Gemäß einem bevorzugten Aspekt kann die Anschlussmaske drei Druckaufnehmeranschlüsse aufweisen, die nach dem vorstehend beschriebenen Poka-Yoke-System an der Blutbehandlungsmaschine angeordnet sind. Besonders bevorzugt kann die Blutbehandlungsmaschine einen arteriellen Druckaufnehmeranschluss, einen venösen Druckaufnehmeranschluss und einen dialysatoreingangsseitigen Druckaufnehmeranschluss aufweisen.

Gemäß einer bevorzugten Weiterbildung dieser Ausführungsform können die drei Druckaufnehmeranschlüsse dreiecksförmig verteilt zueinander beabstandet auf der Anschlussmaske angeordnet sein. Hierbei können bevorzugt die Kantenlängen des so gebildeten Dreiecks (der Abstand jedes Druckaufnehmeranschlusses zu den beiden anderen Druckaufnehmeranschlüssen) so gewählt sein, dass sie größer als die Schlauchlänge der längsten der drei Drucküberwachungsleitungen des Schlauchsets sind. Bevorzugt können diese Kantenlängen/Abstände in einem Bereich von 10 bis 100 cm, weiter bevorzugt zwischen 20 und 50 cm gewählt sein (bspw. 30cm). Die dreiecksförmige Anordnung ermöglich eine gute Ausnutzung der begrenzt zur Verfügung stehenden Fläche.

Gemäß einem bevorzugten Ausführungsbeispiel der Offenbarung kann einer der Druckaufnehmeranschlüsse an der Anschlussmaske in Vertikalrichtung der Blutbehandlungsmaschine gesehen tieferliegend angeordnet sein als der zumindest eine andere Druckaufnehmeranschluss. Insbesondere kann der Druckaufnehmeranschluss um eine Distanz größer oder gleich der Länge der zugehörigen Drucküberwachungsleitung bevorzugt um eine Distanz in einem Bereich zwischen 20 und 70 cm.

Gemäß einer bevorzugten Ausführungsform kann einer der Druckaufnehmeranschlüsse in Vertikalrichtung der Blutbehandlungsmaschine gesehen tieferliegend als die Fördervorrichtung angeordnet sein. Insbesondere kann der arterielle Druckaufnehmeranschluss in einem Bereich nahe einem Eingang/Einlass der Fördervorrichtung angeordnet sein. Bevorzugt ist hierbei ein Abstand kleiner als 30 cm, besonders bevorzugt kleiner 15 cm, zum Eingang der Fördervorrichtung.

Vorzugsweise kann der dialysatoreingangsseitige Druckaufnehmeranschluss in einem Bereich nahe einem Ausgang/Auslass der Fördervorrichtung angeordnet sein. Bevorzugt ist hierbei ein Abstand kleiner als 30 cm, besonders bevorzugt kleiner 15 cm, zum Ausgang der Fördervorrichtung.

Bevorzugt kann der venöse Druckaufnehmeranschluss in einem Bereich oberhalb eines Halters für einen Entlüfter (eine Luftfalle) der Anschlussmaske angeordnet sein. Bevorzugt ist hierbei ein Abstand kleiner als 30 cm, besonders bevorzugt kleiner 15 cm, zum Ausgang der Fördervorrichtung.

In einer bevorzugten Ausgestaltung der Offenbarung ist die Fördervorrichtung eine Schlauchpumpe (auch Schlauchquetschpumpe, Rollen-, Rollerpumpe oder Peristaltikpumpe genannt).

Weiter bevorzugt sind die (alle) Druckaufnehmeranschlüsse als, vorzugsweise identische, Konnektoren, insbesondere Luer-Lock-Konnektoren, ausgebildet, welche vorzugsweise eingerichtet sind, um mit an den Drucküberwachungsleitungen vorgesehenen Konnektoren, insbesondere Luer-Lock-Konnektoren, verbunden zu werden. Weiter bevorzugt sind die Druckaufnehmeranschlüsse als Gehäusedurchführungen mit gehäuseaußenseitigem Luer-Lock-Konnnektor ausgeführt.

Ein weiterer Aspekt der Offenbarung betrifft ein Schlauchset zur Verwendung mit einer extrakorporalen Blutbehandlungsmaschine, insbesondere gemäß einem der vorgenannten Aspekte, das lösbar/austauschbar und in einer vorbestimmten Anordnung an einer Anschlussmaske der Blutbehandlungsmaschine anbringbar ist. Das Schlauchset hat zumindest eine Blutzuführleitung zum Zuführen von Blut zu einer Blutbehandlungseinheit, eine Blutrückführleitung zum Rückführen von behandeltem Blut von der Behandlungseinheit, einen Pumpenabschnitt, der dazu angepasst und vorgesehen ist, in eine Fördervorrichtung der extrakorporalen Blutbehandlungsmaschine eingelegt zu werden und mit dieser zusammenzuwirken, sowie zumindest zwei Drucküberwachungsleitungen, die jeweils an einer Abzweigung von der Blutzuführleitung oder der Blutrückführleitung abgehen und mit an der Blutbehandlungsmaschine angeordneten Druckaufnehmeranschlüssen verbindbar sind, um einen Druck von den Abzweigungen zu einem Druckaufnehmeranschluss zu übertragen. Die Länge der Drucküberwachungsleitungen sowie die Positionierung der Abzweigungen (in Längserstreckungsrichtung der Blutzuführleitung und der Blutrückführleitung) sind derart angepasst, dass die Drucküberwachungsleitungen, wenn das Schlauchset in der vorbestimmten Anordnung an der Anschlussmaske angebracht ist, jeweils nur das Herstellen einer Verbindung mit einem vorbestimmten Druckaufnehmeranschluss aus der Anzahl an Druckaufnehmeranschlüssen zulassen.

Auf diese Weise lässt sich ein Poka-Yoke-System für die Drucküberwachungsleitungen implementieren, dass die Bedienung der Maschine vereinfacht und Montagezeiten für das Anbringen des Schlauchsets reduziert.

Gemäß einem bevorzugten Ausführungsbeispiel der Offenbarung kann jede der Drucküberwachungsleitungen an ihrem vom Schlauchset abgewandten Ende einen identischen Anschluss, insbesondere einen Luer-Lock-Konnektor, zum Anschließen an den vorbestimmten Druckaufnehmeranschluss aufweisen. Da eine Verwechslung bereits durch die angepassten Schlauchlängen ausgeschlossen ist, können die Anschlüsse für Druckaufnehmer zur Vereinfachung der Herstellung identisch gehalten werden.

Gemäß einem weiter bevorzugten Aspekt kann der Pumpenabschnitt in der Blutzuführleitung ausgebildet sein und die Blutzuführleitung kann in Förderrichtung dem Pumpenabschnitt vorgelagert eine arterielle Abzweigung aufweisen, von der eine arterielle Drucküberwachungsleitung abgeht, die an einen arteriellen Druckaufnehmeranschluss der Dialysemaschine anschließbar ist.

Gemäß einem weiter bevorzugten Aspekt kann die dialysatoreingangsseitige Abzweigung zwischen dem Pumpenabschnitt und einem Anschlussstück zum Anschließen der Blutzuführleitung an einen Eingang des Dialysators angeordnet sein.

Gemäß einem weiter bevorzugten Aspekt kann die Blutrückführleitung zwischen einem Anschlussstück zum Anschließen an einen Ausgang des Dialysators und einer Luftfalle angeordnet eine venöse Abzweigung aufweisen, von der eine venöse Drucküberwachungsleitung abgeht, die an einen venösen Druckaufnehmeranschluss der Dialysemaschine anschließbar ist.

Gemäß einer bevorzugten Ausführungsform kann zumindest eine der Abzweigungen als separates Abzweigungselement in einem Schlauchabschnitt eingefügt sein, d.h. nicht aus einem Entlüfter oder einer vergleichbaren Komponente abgehen. Insbesondere können alle Abzweigungen, als ein solches separates Abzweigungselement in einem Schlauchabschnitt eingefügt sein. Dies ermöglicht eine flexiblere Gestaltung der Anordnung der Abzweigungen.

Vorzugsweise können alle Drucküberwachungsschläuche mit einer Schlauchlänge kürzer als 100 cm ausgeführt sein. Besonders bevorzugt können Drucküberwachungsschläuche in einem Längenbereich zwischen 10 und 50 cm ausgebildet sein.

Gemäß einem bevorzugten Ausführungsbeispiel der Offenbarung kann zumindest eine der Abzweigungen als ein (separates) Abzweigungselement mit einer oszillierenden Druckmembran ("pressure oscillating diaphragm" kurz: POD) ausgebildet sein. Ein POD ist ein Abzweigungselement, dass die Abzweigung mittels einer Membran fluidisch abdichtet bzw. vom extrakorporalen Blutkreislauf trennt über die Flexibilität der Membran aber Druckübertragung zulässt. Insbesondere können alle Abzweigungen als Abzweigungselemente mit derartigen PODs ausgebildet sein.

Gemäß einem ggf. unabhängig zu beanspruchenden weiteren Aspekt der Offenbarung kann die zumindest eine Abzweigung, in einem vorbestimmten Winkel, insbesondere einem Winkel zwischen 45° und 90°, relativ zum zugehörigen Schlauchabschnitt der Blutzuführleitung oder der Blutrückführleitung abgehen. Dies kann bevorzugt so ausgeführt sein, dass, wenn das Schlauchset in der vorbestimmten Anordnung an der Anschlussmaske montiert ist, die Abzweigung im Wesentlichen in Richtung hin zum zugehörigen Druckaufnehmeranschluss an der Blutbehandlungsmaschine orientiert ist. Bevorzugt kann der zugehörige Druckaufnehmeranschluss von der Abzweigungsrichtung der Abzweigung aus gesehen in einem Winkelbereich/Kegel von ± 90°, bevorzugt ±45°, besonders bevorzugt ±15°, befinden. Auf diese Weise kann bereits die Orientierung der Abzweigung für die Drucküberwachungsleitungen den Anwender beim korrekten Verbinden mit den Druckaufnehmeranschlüssen unterstützen.

Gemäß einem weiter bevorzugten Aspekt weisen die (alle) Drucküberwachungsleitungen an ihrem der Abzweigung abgewandtem Ende, vorzugsweise identische, Anschlüsse bzw. Konnektoren, insbesondere Luer-Lock-Konnektoren, auf, welche bevorzugt eingerichtet bzw. vorbereitet sind, um mit den Druckaufnehmeranschlüssen verbunden zu werden.

Die Erfindung betrifft ein System aus einer extrakorporalen Blutbehandlungsmaschine und einem Schlauchset, die gemäß einem oder mehreren der vorstehend für die einzelnen Komponenten beschriebenen Aspekte, aufeinander abgestimmt sind.

Insbesondere betrifft die Erfindung also ein System aus einer extrakorporalen Blutbehandlungsmaschine, insbesondere wie voranstehend beschrieben, und einem lösbar bzw. austauschbar und in einer vorbestimmten Anordnung an der Blutbehandlungsmaschine anbringbaren Schlauchset, insbesondere wie voranstehend beschrieben, wobei die extrakorporale Blutbehandlungsmaschine, insbesondere Dialysemaschine, eine Blutbehandlungsvorrichtung, insbesondere Dialysator-Filterkartusche, eine Fördervorrichtung zum Fördern von Blut durch die Blutbehandlungsvorrichtung, und eine Anschlussmaske, an welcher Druckaufnehmeranschlüsse angeordnet sind, enthält, wobei das Schlauchset zumindest eine Blutzuführleitung zum Zuführen von Blut zur Blutbehandlungsvorrichtung, eine Blutrückführleitung zum Rückführen von behandeltem Blut von der Blutbehandlungsvorrichtung, einen Pumpenabschnitt, der dazu angepasst und vorgesehen ist, in die Fördervorrichtung der extrakorporalen Blutbehandlungsmaschine eingelegt zu werden, sowie zumindest zwei Drucküberwachungsleitungen, die jeweils an einer Abzweigung von der Blutzuführleitung oder der Blutrückführleitung abgehen, enthält, wobei die Drucküberwachungsleitungen mit den Druckaufnehmeranschlüssen verbindbar sind, um einen Druck von den Abzweigungen zum entsprechenden Druckaufnehmeranschluss zu übertragen, und wobei die Druckaufnehmeranschlüsse derart voneinander beabstandet und in ihrer Positionierung an der extrakorporalen Blutbehandlungsmaschine auf das Schlauchset abgestimmt sind, und die Länge der Drucküberwachungsleitungen sowie die Positionierung der Abzweigungen derart angepasst sind, dass, wenn das Schlauchset in der vorbestimmten Anordnung an der Anschlussmaske montiert ist, jede Drucküberwachungsleitung aufgrund ihrer begrenzten Länge und der vorbestimmten Anordnung der zugehörigen Abzweigung an der Blutbehandlungsmaschine ausschließlich mit je einem der Druckaufnehmeranschlüsse verbindbar ist.

Gemäß einem Aspekt eines solchen Systems können die Drucküberwachungsschlauch-Längen und die Lage der Abzweigungen in der an der Anschlussmaske befestigten Anordnung des Schlauchsets derart gewählt sein, dass der Drucküberwachungsschlauch gerade zum vorbestimmten Druckaufnehmeranschluss reicht plus ein bestimmtes Übermaß, das ein besseres Handling ermöglicht. Bevorzugt kann dieses Übermaß kleiner als 20 cm, insbesondere kleiner als 10 cm, besonders bevorzugt kleiner als 5 cm, gewählt sein.

### Kurzbeschreibung der Figuren

Die vorliegende Offenbarung wird nachstehend anhand eines in den Figuren dargestellten bevorzugten Ausführungsbeispiels beschrieben.
Fig. 1 ist eine Darstellung zur Veranschaulichung eines Systemaufbaus gemäß einem ersten Ausführungsbeispiel der vorliegenden Offenbarung;
Fig. 2 ist eine Darstellung zur Veranschaulichung des Systemaufbaus gemäß dem ersten Ausführungsbeispiel mit eingezeichneten Radien der Drucküberwachungsleitungen;
Fig. 3 ist eine Schnittansicht eines ersten oszillierenden Druckmembran Anschlusses (POD);
Fig. 4 ist eine perspektivische Ansicht des ersten oszillierenden Druckmembran Anschlusses (POD);
Fig. 5 ist eine schematische Ansicht eines zweiten oszillierenden Druckmembran Anschlusses (POD) im eingebauten Zustand;
Fig. 6 ist eine schematische Ansicht des zweiten oszillierenden Druckmembran Anschlusses (POD);
Fig.7 ist eine perspektivische Ansicht eines dritten oszillierenden Druckmembran Anschlusses (POD);
Fig. 8 ist eine Darstellung eines beispielhaften Schlauchsets gemäß der vorliegenden Offenbarung; und
Fig. 9 zeigt eine Anschlussmaske einer Dialysemaschine aus dem Stand der Technik mit daran angebrachtem Schlauchset.

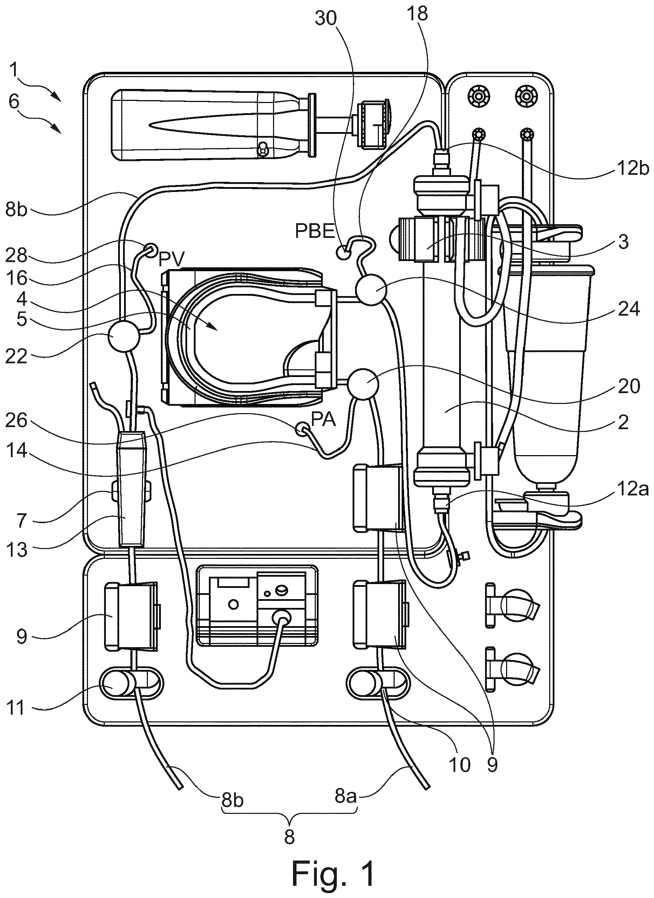

Fig. 1 zeigt schematisch eine Dialysemaschine 1 gemäß einem bevorzugten Ausführungsbeispiel der Offenbarung in einer Frontansicht. Die Dialysemaschine 1 weist an ihrer in der Fig. 1 dargestellten Gehäusefront eine Blutbehandlungsvorrichtung 2 (hier einen Dialysator) auf, die in einer vorbestimmten Position und Ausrichtung austauschbar durch eine Halterung 3 (hier eine Dialysatorhalterung) gehalten wird. An der Gehäusefront ist zusätzlich eine Fördervorrichtung 4 bzw. eine Schlauchpumpe/Peristaltikpumpe vorgesehen, die dazu ausgebildet ist, Blut über einen extrakorporalen Blutkreislauf durch den Dialysator 2 zu pumpen. Besagter extrakorporaler Blutkreislauf wird durch ein an einer Anschlussmaske/Anschlussstruktur 6 der Dialysemaschine auswechselbar gehaltenes Schlauchset 8 (Einmalartikel) gebildet. Die Anschlussmaske 6 wird durch verschiedene Komponenten an der Gehäusefront der Dialysemaschine 1 ausgebildet, die spezifisch auf das Schlauchset 8 angepasst sind und mittels welcher das Schlauchset 8 auf definierte Art und Weise lösbar an der Gehäusefront anbringbar ist, sodass es mit der Fördervorrichtung 4 und dem Dialysator 2 angeschlossen bzw. wirkverbunden werden kann. Die Fördervorrichtung 4 (Schlauchquetschpumpe) kann als Teil der Anschlussmaske 6 angesehen werden, da bei eingelegtem Schlauchset 8 die Position eines Pumpenabschnitts 5 des Schlauchsets 8 festgelegt wird. Der Pumpenabschnitt 5 ist ein verstärkt ausgeführter Schlauchabschnitt, der für die Wechselbelastung durch die Fördervorrichtung 4 ausgelegt ist. Weitere Komponenten der Anschlussmaske 6 sind u.a. die (Dialysator-)Halterung 3, eine Entlüfterhalterung 7, diverse Sensoren 9 (bspw. Temperatursensor, Hematokritsensor, Luftdetektor oder dergleichen), eine arterielle Schlauchklemme 10 und eine venöse Schlauchklemme 11. Allen diesen Komponenten, die unter dem Begriff Anschlussmaske 6 zusammengefasst werden, gemein ist, dass Abschnitte des Schlauchsets 8 für den Betrieb daran angeschlossen oder in diese eingelegt werden, wodurch die Komponenten eine charakteristische Anordnung des Schlauchsets 8 bzw. den Verlauf des extrakorporalen Blutkreislaufes an der Dialysemaschine vorgeben.

Zusammengefasst bildet das Schlauchset 8 in der in Fig. 1 dargestellten bevorzugten Ausführungsform zusammen mit einer nicht näher dargestellten Blutkammer des Dialysators 2 einen extrakorporalen Blutkreislauf aus, durch welchen mittels der Fördervorrichtung 4 Blut zirkuliert werden kann. Schlauchset 8 und Dialysator 2 sind an der Anschlussmaske 6 der Dialysemaschine 1 angebracht, die eine vorbestimmte Anordnung an der Dialysemaschine 1 vorgibt. Schlauchset 8 und Dialysator 2 sind aufgrund des während einer Behandlung entstehenden direkten Blutkontaktes als austauschbare Einweg-Artikel ausgeführt, während Anschlussmaskenkomponenten wie die Fördervorrichtung 4 fest in der Gehäusefront installiert sind.

Figur 1 zeigt das Schlauchset 8 gemäß der bevorzugten Ausführungsform, wie es in der vorbestimmten Anordnung an der Anschlussmaske 6 der Dialysemaschine 1 angebracht ist. Das Schlauchset 8 lässt sich grob in eine Blutzuführleitung 8a (bzw. arteriellen Schlauchabschnitt) zum Zuführen von Blut eines Patienten zum Dialysator 2 und eine Blutrückführleitung 8b (bzw. venösen Schlauchabschnitt) zum Rückführen von behandeltem Blut von der Behandlungseinheit 2 zum Patienten unterteilen. Das in Fig. 1 abgebildete Schlauchset 8 weist weiter ein Eingangs-Anschlussstück 12a zum Anschließen der Blutzuführleitung 8a an einen Eingang der Blutkammer des Dialysators 2, ein Ausgangs-Anschlussstück 12b zum Anschließen der Blutrückführleitung 8b an einen Ausgang der Blutkammer des Dialysators 2 sowie eine in der Blutrückführleitung 8b angeordnete Luftfalle bzw. einen Entlüfter 13 zum Herausfiltern von Luftblasen aus dem rückströmenden Blut auf.

Zum Gewährleisten der Patientensicherheit sowie optimaler Betriebsparameter, müssen die blutführenden Leitungen des Schlauchsets 8 drucküberwacht werden. Zu diesem Zweck gehen Drucküberwachungsleitungen 14, 16, 18 an definierten Abzweigungen 20, 22, 24 von der Blutzuführleitung 8a und der Blutrückführleitung 8b ab. Besagte Drucküberwachungsleitungen 14, 16, 18 sind an maschinenseitige Druckaufnehmeranschlüsse 26, 28, 30 angeschlossen, an welche innerhalb der Dialysemaschine angeordnete und deshalb nicht näher dargestellte Druckaufnehmer bzw. Drucksensoren angeschlossen sind. Die Druckaufnehmeranschlüsse 26, 28, 30 sind in der bevorzugten Ausführungsform als Gehäusedurchführungen mit gehäuseaußenseitigem Luer-Lock-Konnektor ausgeführt.

Im Detail weist in der in Figur 1 dargestellten Anordnung die Blutzuführleitung 8a in Förderrichtung (im Normalbetrieb) der Fördervorrichtung 4 vorgelagert eine arterielle Abzweigung 20 auf, von der eine arterielle Drucküberwachungsleitung 14 abgeht, die an einen arteriellen Druckaufnehmeranschluss 26 der Dialysemaschine 1 angeschlossen ist und den Unterdruck (PA) am zum arteriellen Patientenzugang hingewandten Schlauchabschnitt überwacht.

Weiter weist in Figur 1 die Blutzuführleitung 8a zwischen Fördervorrichtung 4 und dem Eingangs-Anschlussstück 12a zum Anschließen an den Eingang des Dialysators 2 eine dialysatoreingangsseitige Abzweigung 24 auf. Von der dialysatoreingangsseitigen Abzweigung 24 geht eine dialysatoreingangsseitige Drucküberwachungsleitung 18 ab. Diese ist an einen dialysatoreingangsseitigen Druckaufnehmeranschluss 30 der Dialysemaschine 1 angeschlossen und überwacht den Druck am Dialysatoreingang (PBE).

Die Blutrückführleitung weist in Figur 1 eine venöse Abzweigung 22 auf, von der eine venöse Drucküberwachungsleitung 16 abgeht, die an einen venösen Druckaufnehmeranschluss 28 der Dialysemaschine 1 angeschlossen ist und den Druck (PV) am venösen Patientenzugang überwacht. Die venöse Abzweigung 22 ist im dargestellten Ausführungsbeispiel zwischen dem Ausgangs-Anschlussstück 12b zum Anschließen der Blutrückführleitung 8b an einen Ausgang der Blutkammer des Dialysators 2 und dem Entlüfter 13 angeordnet.

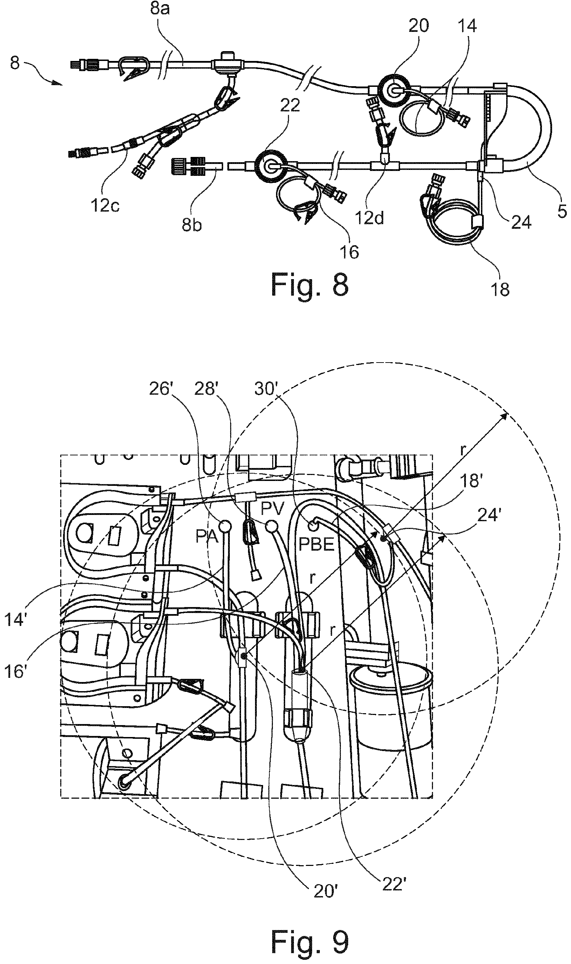

In der Figur 9 ist beispielhaft ein Ausschnitt einer Front einer Dialysemaschine 1', wie sie aus dem Stand der Technik bekannt ist, dargestellt. Die Dialysemaschine 1' weist zum Großteil ähnliche Komponenten wie die vorstehend beschriebene Dialysemaschine 1 gemäß dem bevorzugten Ausführungsbeispiel auf. Die Drucküberwachungsleitung 14', 16', 18' ist im Stand der Technik bspw. über ein T-Stück mit dem Schlauch 8a', 8b' verbunden oder geht direkt vom Entlüfter 13' oder vergleichbaren Komponenten mit einem stehenden Luftvolumen ab. Zwischen der Drucküberwachungsleitung 14', 16', 18' und dem Druckaufnehmer befindet sich dann ein Luftpolster, welches sich bei Druckänderung im Schlauch 8a', 8b` in seinem Volumen verändert, was wiederum zu einer Auslenkung des Druckaufnehmers führt. Ein derartiges Druckmessverfahren bringt unter anderem den Nachteil mit sich, dass der Druckaufnehmeranschluss 26', 28', 30' immer oberhalb der Abzweigung 20', 22', 24' angeordnet sein muss (eine Luftsäule zwischen der Abzweigung und dem Druckaufnehmer vorhanden sein muss). Ansonsten besteht die Gefahr eines direkten Kontaktes des Drucksensors/Druckaufnehmers mit der Flüssigkeit.

Wie in Fig. 9 gut zu erkennen ist, liegen die Druckaufnehmeranschlüsse 26', 28', 30' dicht beieinander in einem oberen Bereich der Anschlussmaske 6. Dies hat zum einen den Hintergrund, dass die Druckaufnehmeranschlüsse 26', 28', 30' wie vorstehend erläutert oberhalb der zugehörigen Abzweigungen 20', 22', 24' platziert sein müssen und zum anderen ist der Großteil der Elektronikkomponenten der Dialysemaschine 1' im oberen Bereich des Maschineninneren gebündelt. Wie in der Fig. 9 durch die Radien r angezeichnet ist, sind die Drucküberwachungsleitungen 14', 16', 18' aufgrund der vergleichsweise tiefen Anordnung der Abzweigungen 20', 22', 24' relativ lang ausgeführt. Dies hat zur Folge, dass die Schläuche 14', 16', 18'beim Anschließen an die Druckaufnehmeranschlüsse 26`, 28`, 30' beliebig vertauscht werden können, da innerhalb jedes durch die Radien r definierten Kreises alle drei Druckaufnehmeranschlüsse 26', 28', 30' liegen.

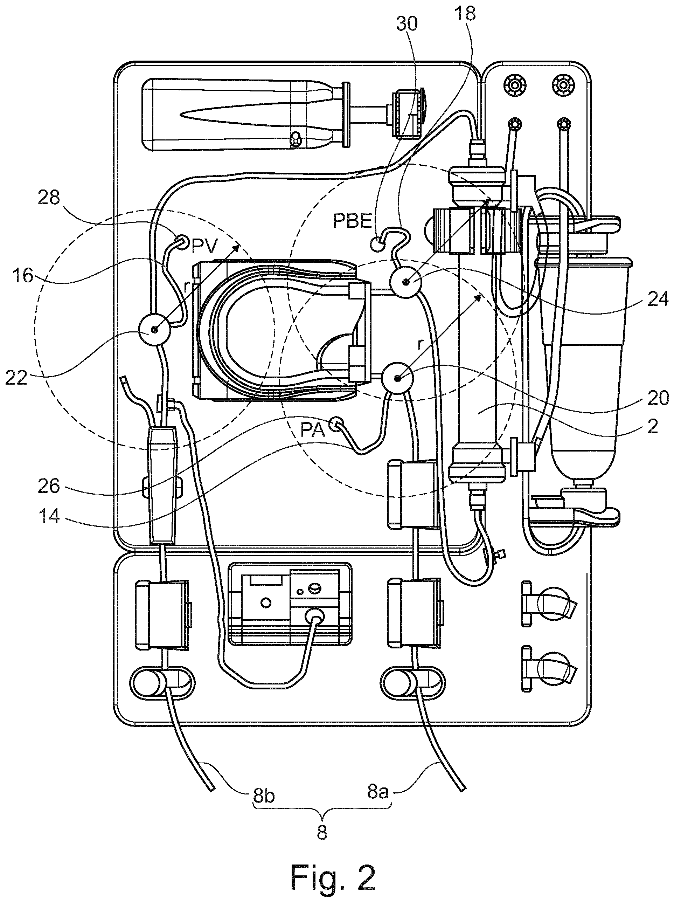

In der Dialysemaschine 1 gemäß dem bevorzugten Ausführungsbeispiel ist, wie am besten in Fig. 2 zu erkennen ist, ein Poka-Yoke-System implementiert, das das Anschließen der Drucküberwachungsleitungen 14, 16, 18 an die Druckaufnehmeranschlüsse 26, 28, 30 drastisch vereinfacht. Hierzu sind die Anschlussmaske 6 und das Schlauchset 8 derart konstruktiv aneinander angepasst, dass in der durch die Anschlussmaske 6 vorgegebenen, vorbestimmten Anordnung des Schlauchsets 8 die Drucküberwachungsleitungen 14, 16, 18 nicht mit einem falschen Druckaufnehmeranschluss 26, 28, 30 verbunden werden können. Im dargestellten Beispiel sind zu diesem Zweck die Abstände zwischen den Druckaufnehmeranschlüssen 26, 28, 30 ausreichend groß gewählt, die Längen der Drucküberwachungsleitungen 14, 16, 18 ausreichend klein gewählt. Zudem ist die Position der Abzweigungen 20, 22, 24 in der vorbestimmten Anordnung des Schlauchsets 8 an der Anschlussmaske 6 so gewählt, dass je nur ein Druckaufnehmeranschluss 26, 28, 30 in Reichweite der von der Abzweigung abgehenden Drucküberwachungsleitungen 14, 16, 18 liegt. Wenn das Schlauchset 8 in der vorbestimmten Anordnung an der Anschlussmaske 6 angeordnet ist, wird also nur das Verbinden der Drucküberwachungsleitungen 14, 16, 18 mit dem jeweils richtigen Druckaufnehmeranschluss 26, 28, 30 zugelassen. In dem in Fig. 2 dargestellten, bevorzugten Ausführungsbeispiel sind alle Drucküberwachungsleitungen 14, 16, 18 mit gleicher Länge ausgebildet. Gemäß einer alternativen bevorzugten Ausführungsform können die Schlauchlängen der Drucküberwachungsleitungen 14, 16, 18 auch deutlich unterschiedlich zueinander sein. Durch die vorgegebene Schlauchlänge ergibt sich für jede Drucküberwachungsleitung 14, 16, 18 ein Radius r, innerhalb dessen in der offenbarungsgemäßen Anordnung immer genau ein Druckaufnehmeranschluss 26, 28, 30 erreichbar ist. Die Schlauchlänge der Drucküberwachungsleitungen 14, 16, 18 ist im dargestellten Beispiel immer so gewählt, dass sie gerade bis zum gewünschten Druckaufnehmeranschluss 26, 28, 30 reicht, plus einen möglichst kurzen Anteil, um ein gutes Handling zu ermöglichen.

In Figur 2 ist gut zu erkennen, dass die Druckaufnehmeranschlüsse 26, 28, 30 dreiecksförmig auf der Gehäusefront der Dialysemaschine 1 verteilt sind. Jede der Kantenlängen dieser Dreiecksform ist dabei größer als die Schlauchlänge der Drucküberwachungsleitungen 14, 16, 18 (hier 30 cm) gewählt. Die dreiecksförmige Anordnung/Verteilung der Druckaufnehmeranschlüsse 26, 28, 30 ermöglicht eine gute Raumausnutzung der begrenzten Fläche im Bereich der Anschlussmaske 6.

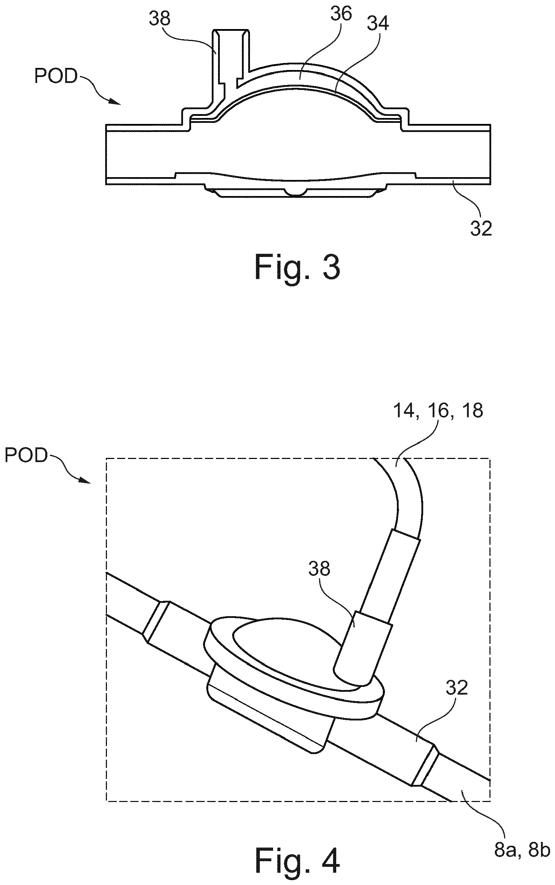

Ein synergistischer Effekt mit der vorstehend beschriebenen Poka-Yoke-Anordnung ergibt sich durch die Verwendung von Abzweigungen mit oszillierenden Druckmembranen auch POD (Pressure Oscillating Diaphragm) genannt. Dabei erfolgt die Drucküberwachung nicht direkt von beispielsweise Blut auf das Luftpolster, sondern Blut und Luft sind über eine flexible Membran voneinander getrennt. Dieser Aufbau ist in Fig. 3 gut zu erkennen, die eine beispielhafte POD im Querschnitt zeigt. Es ist zu erkennen, dass ein Blutleitungsabschnitt 32 des PODs, der als Zwischenstück in die Schlauchleitungen 8a, 8b einsetzbar ist, und eine Luftkammer 36 durch eine flexible, fluiddichte Membran 34 getrennt sind. Ein Konnektor 38 dient zum Anschließen einer Drucküberwachungsleitungen 14, 16, 18 mit der Luftkammer 36. Durch eine Druckänderung in dem Blutleitungsabschnitt 32 wird die Membran 34 ausgelenkt und diese Krafteinwirkung wird über ein Luftpolster auf einen Drucksensor übertragen, welcher den Schlauchinnendruck misst. Dieser Messaufbau hat den Vorteil, dass Blut und Luftsäule entkoppelt werden, sodass der Druckaufnehmeranschluss nicht mehr oberhalb der Abzweigung angeordnet werden muss. Folglich ergibt sich eine größere Gestaltungsfreiheit bei der Positionierung sowohl der Abzweigungen 20, 22, 24 (im montierten Zustand des Schlauchsets 8) als auch der Druckaufnehmeranschlüsse 26, 28, 30. Ein positiver Nebeneffekt ist, dass der Blut-Luft-Kontakt verringert wird, was die Blutgerinnung reduziert.

Durch die vorstehende Verwendung von PODs kann in dem in Figur 1 dargestellten Beispiel der arterielle Druckaufnehmeranschluss 26 vertikal unterhalb der Fördervorrichtung 4 platziert werden (während die anderen Druckaufnehmeranschlüsse 28, 30 oberhalb der Fördervorrichtung angeordnet sind). Hierdurch ergibt sich eine günstige Beabstandung zu den verbleibenden Druckaufnehmeranschlüssen 28, 30.

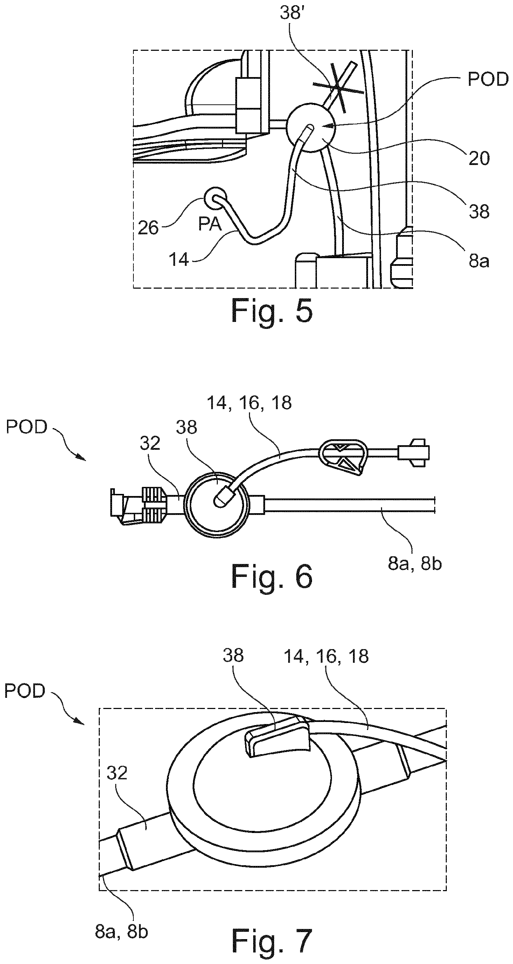

Figuren 4, 6 und 7 zeigen verschiedene Ausführungsformen von PODs, bei denen der Konnektor 38 jeweils in anderen Ausrichtungen von der Luftkammer 36 abgeht. In handelsüblichen PODs ist der Konnektor 38 meist senkrecht zur POD Kreisfläche bzw. zur Membran 34 ausgerichtet (vgl. Figur 4) oder verläuft parallel zum Blutleitungsabschnitt 32 (vgl. Figur 7).

In der bspw. in Figur 1 dargestellten bevorzugten Ausführungsform der Offenbarung weist das Schlauchset ausschließlich als PODs ausgebildete Abzweigungen 20, 22, 24 auf. Bei besagten PODs 20, 22, 24 ist der Konnektor 38 jeweils parallel zur POD Kreisfläche (zur Gehäusefront) aber in einem definierten Winkel (in der Draufsicht) zum Blutleitungsabschnitt 32 ausgerichtet. Eine solche POD Abzweigung ist auch in Figur 6 im Detail dargestellt. In dem in Figur 1 dargestellten Ausführungsbeispiel sind die Abgangswinkel der Konnektoren 38 der PODs derart konfiguriert, dass, wenn das Schlauchset 8 in der vorbestimmten Anordnung an der Anschlussmaske angebracht ist, die Konnektoren 38 bereits zum zugehörigen Druckaufnehmeranschluss 26, 28, 30 hin ausgerichtet sind. Der Anwender wird dadurch bei der Montage des Schlauchsets auf intuitiver Ebene unterstützt.

Dieses Konzept ist in der Figur 5 nochmals detailliert dargestellt. Wie ebenfalls durch den durchgestrichenen Konnektor 38' in Figur 5 symbolisiert ist, kann auf diese Weise in jedem Fall verhindert werden, dass die Abzweigungen 20, 22, 24 entgegengesetzt zum vorgesehenen Druckaufnehmeranschluss 26, 28, 30 zeigen.

In anderen Worten kann die Orientierung aller Abzweigungen 20, 22, 24 also bevorzugt so gewählt sein, dass sie um weniger als 90° (besser weniger als 15°) von einer direkten Luftlinie zum vorgesehenen Druckaufnehmeranschluss 26, 28, 30 abweichen.

Figur 8 zeigt ein Schlauchset 8 gemäß einer weiteren Ausführungsform der Offenbarung in einer isolierten Darstellung. Das in Fig. 8 abgebildete Schlauchset 8 weist eine als POD ausgebildete arterielle Abzweigung 20 und eine als POD ausgebildete venöse Abzweigung 22 auf. Die dialysatoreingangsseitige Abzweigung 24 mit der zugehörigen Drucküberwachungsleitung geht im Schlauchset gemäß Fig. 8 direkt einem Einsatzstück des Pumpenabschnitts 5 ab. In der zugehörigen Dialysemaschine (nicht dargestellt) liegt der dialysatoreingangsseitige Druckaufnehmeranschluss 30 vertikal oberhalb der Fördervorrichtung 4, sodass die Abzweigung 24 in dessen Richtung orientiert ist. Die von diesen abgehenden Drucküberwachungsleitungen 14, 16 haben eine Schlauchlänge von 20 cm und je einen Luer-Lock-Konnektor an ihrem freien Ende. Das Eingangs-Anschlussstück 12a zum Anschließen der Blutzuführleitung 8a an einen Eingang der Blutkammer des Dialysators 2, das Ausgangs-Anschlussstück 12b zum Anschließen der Blutrückführleitung 8b an einen Ausgang der Blutkammer des Dialysators 2 und der Entlüfter 13 sind in der Figur 8 der Übersichtlichkeit halber ausgeblendet. In dem Schlauchset 8 der Figur 8 ist zusätzlich ein Prädilutions-Anschlussstück 12c in der Blutzuführleitung 8a und ein Prädilutions-Anschlussstück 12d in der Blutrückführleitung 8b vorgesehen.

### Bezugszeichen

- 1: Extrakorporale Blutbehandlungsmaschine / Dialysemaschine;
- 2: Blutbehandlungsvorrichtung / Filterkartusche;
- 3: Dialysatorhalterung;
- 4: Fördervorrichtung / Schlauchpumpe;
- 5: Pumpenabschnitt der Blutzuführleitung;
- 6: Anschlussmaske;
- 7: Entlüfterhalterung;
- 8: Schlauchset;
- 8a: Blutzuführleitung;
- 8b: Blutrückführleitung;
- 9: Sensor;
- 10: arterielle Schlauchklemme;
- 11: venöse Schlauchklemme;
- 12a: Eingangs-Anschlussstück;
- 12b: Ausgangs-Anschlussstück;
- 12c: Prädilutions-Anschlussstück;
- 12d: Postdilutions-Anschlussstück;
- 13: Luftfalle / Entlüfter;
- 14: arterielle Drucküberwachungsleitung;
- 16: venöse Drucküberwachungsleitung;
- 18: dialysatoreingangsseitige Drucküberwachungsleitung;
- 20: arterielle Abzweigung / POD;
- 22: venöse Abzweigung / POD;
- 24: dialysatoreingangsseitige Abzweigung / POD;
- 26: arterielle Druckaufnehmeranschluss / PA;
- 28: venöse Druckaufnehmeranschluss / PV;
- 30: dialysatoreingangsseitige Druckaufnehmeranschluss / PBE
- 32: Blutleitungsabschnitt;
- 34: Membran;
- 36: Luftkammer;
- 38: Konnektor;
- POD: Abzweigungselement mit oszillierender Druckmembran; und
- r: Radius der Drucküberwachungsleitung.

## Patentansprüche

1. System aus einer extrakorporalen Blutbehandlungsmaschine (1) und einem lösbar bzw. austauschbar und in einer vorbestimmten Anordnung an der Blutbehandlungsmaschine (1) anbringbaren Schlauchset (8), wobei
die extrakorporale Blutbehandlungsmaschine (1) eine Blutbehandlungsvorrichtung (2), eine Fördervorrichtung (4) zum Fördern von Blut durch die Blutbehandlungsvorrichtung (2), und eine Anschlussmaske (6), an welcher Druckaufnehmeranschlüsse (26, 28, 30) angeordnet sind, enthält; wobei
das Schlauchset (8) zumindest eine Blutzuführleitung (8a) zum Zuführen von Blut zur Blutbehandlungsvorrichtung (2), eine Blutrückführleitung (8b) zum Rückführen von behandeltem Blut von der Blutbehandlungsvorrichtung (2), einen Pumpenabschnitt (5), der dazu angepasst und vorgesehen ist, in die Fördervorrichtung (4) der extrakorporalen Blutbehandlungsmaschine (1) eingelegt zu werden, sowie zumindest zwei Drucküberwachungsleitungen (14, 16, 18), die jeweils an einer Abzweigung (20, 22, 24) von der Blutzuführleitung (8a) oder der Blutrückführleitung (8b) abgehen, enthält; wobei
die Drucküberwachungsleitungen (14, 16, 18) mit den Druckaufnehmeranschlüssen (26, 28, 30) verbindbar sind, um einen Druck von den Abzweigungen (20, 22, 24) zum entsprechenden Druckaufnehmeranschluss (26, 28, 30) zu übertragen,
**dadurch gekennzeichnet, dass**
die Druckaufnehmeranschlüsse (26, 28, 30) derart voneinander beabstandet und in ihrer Positionierung an der extrakorporalen Blutbehandlungsmaschine (1) auf das Schlauchset (8) abgestimmt sind, und die Länge der Drucküberwachungsleitungen (14, 16, 18) sowie die Positionierung der Abzweigungen (20, 22, 24) derart angepasst sind, dass, wenn das Schlauchset (8) in der vorbestimmten Anordnung an der Anschlussmaske (6) montiert ist, jede Drucküberwachungsleitung (14. 16, 18) aufgrund ihrer begrenzten Länge und der vorbestimmten Anordnung der zugehörigen Abzweigung (20, 22, 24) an der Blutbehandlungsmaschine ausschließlich mit je einem der Druckaufnehmeranschlüsse (26, 28, 30) verbindbar ist.

2. System gemäß Anspruch 1, **dadurch gekennzeichnet, dass** die Anschlussmaske (6) einen arteriellen Druckaufnehmeranschluss (26), einen venösen Druckaufnehmeranschluss (28) und einen blutbehandlungsvorrichtungeingangsseitigen Druckaufnehmeranschluss (30) aufweist, die zueinander beabstandet und dreiecksförmig verteilt auf der Anschlussmaske (6) angeordnet sind.

3. System gemäß einem der Ansprüche 1 oder 2, **dadurch gekennzeichnet, dass** einer der Druckaufnehmeranschlüsse (26) an der Anschlussmaske (6) in Vertikalrichtung der Blutbehandlungsmaschine (1) gesehen tieferliegend angeordnet ist als der zumindest eine andere Druckaufnehmeranschluss (28, 30), vorzugsweise um eine Distanz größer oder gleich der Länge der zugehörigen Drucküberwachungsleitung (14).

4. System gemäß Anspruch 3, **dadurch gekennzeichnet, dass** einer der Druckaufnehmeranschlüsse (26, 28, 30), vorzugsweise der arterielle Druckaufnehmeranschluss (26), in Vertikalrichtung der Blutbehandlungsmaschine (1) gesehen tieferliegend als die Fördervorrichtung (4) angeordnet ist.

5. System gemäß einem der Ansprüche 1 bis 4, **dadurch gekennzeichnet, dass** die Druckaufnehmeranschlüsse (26, 28, 30) als, vorzugsweise identische, Konnektoren, vorzugsweise Luer-Lock-Konnektoren, ausgebildet sind, welche eingerichtet sind, um mit an den Drucküberwachungsleitungen (14, 16, 18) vorgesehenen Konnektoren, vorzugsweise Luer-Lock-Konnektoren, verbunden zu werden.

6. System gemäß Anspruch 1, **dadurch gekennzeichnet, dass** der Pumpenabschnitt (5) in der Blutzuführleitung (8a) ausgebildet ist und die Blutzuführleitung (8a) in Förderrichtung dem Pumpenabschnitt (5) vorgelagert eine arterielle Abzweigung (20) aufweist, von der eine arterielle Drucküberwachungsleitung (14) abgeht, die an einen arteriellen Druckaufnehmeranschluss (26) der Blutbehandlungsmaschine (1) anschließbar ist und zwischen dem Pumpenabschnitt (5) und einem Anschlussstück (12a) zum Anschließen der Blutzuführleitung (8a) an einen Eingang der Blutbehandlungsvorrichtung (2) eine blutbehandlungsvorrichtungeingangsseitige Abzweigung (24) aufweist, von der eine blutbehandlungsvorrichtungeingangsseitige Drucküberwachungsleitung (18) abgeht, die an einen blutbehandlungsvorrichtungeingangsseitigen Druckaufnehmeranschluss (30) der Blutbehandlungsmaschine (1) anschließbar ist.

7. System gemäß Anspruch 1 oder 6, **dadurch gekennzeichnet, dass** die Blutrückführleitung (8b) zwischen einem Anschlussstück (12b) zum Anschließen an einen Ausgang der Blutbehandlungsvorrichtung (2) und einer Luftfalle (13) eine venöse Abzweigung (22) aufweist, von der eine venöse Drucküberwachungsleitung (16) abgeht, die an einen venösen Druckaufnehmeranschluss (28) der Blutbehandlungsmaschine (1) anschließbar ist.

8. System gemäß Anspruch 1, **dadurch gekennzeichnet, dass** zumindest eine der Abzweigungen (20, 22, 24), vorzugsweise alle Abzweigungen (20, 22, 24), als separates Abzweigungselement mit einer die Abzweigung fluidisch abdichtenden oszillierenden Druckmembran (POD), ausgebildet ist.

9. System gemäß Anspruch 8, **dadurch gekennzeichnet, dass** die zumindest eine Abzweigung (20, 22, 24), in einem vorbestimmten Winkel, vorzugsweise einem Winkel zwischen 45° und 90°, relativ zum zugehörigen Schlauchabschnitt der Blutzuführleitung (8a) oder der Blutrückführleitung (8b) abgeht, sodass, wenn das Schlauchset (8) in der vorbestimmten Anordnung an der Anschlussmaske (6) montiert ist, die Abzweigung (20, 22, 24) in Richtung hin zum zugehörigen Druckaufnehmeranschluss (26, 28, 30) an der Blutbehandlungsmaschine (1) orientiert ist.

10. System gemäß einem der Ansprüche 1 oder 6 bis 9, **dadurch gekennzeichnet, dass** die Drucküberwachungsleitungen (14, 16, 18) an ihrem der Abzweigung (20, 22, 24) abgewandtem Ende, vorzugsweise identische, Anschlüsse bzw. Konnektoren, vorzugsweise Luer-Lock-Konnektoren, aufweisen, welche eingerichtet sind, um mit den Druckaufnehmeranschlüssen (26, 28, 30) verbunden zu werden.

## Claims

1. A system comprising an extracorporeal blood treatment machine (1), and a tube set (8) that can be mounted detachably and, resp., interchangeably and in a predefined arrangement on the blood treatment machine, wherein
the extracorporeal blood treatment machine (1), comprises a blood treatment device (2) a conveying device (4) for conveying blood through the blood treatment device (2), and a connection mask (6) on which pressure sensor connections (26, 28, 30) are located; wherein
the tube set (8) comprises at least one blood supply line (8a) for supplying blood to the blood treatment device (2), one blood return line (8b) for returning treated blood from the blood treatment device (2), one pump section (5) adapted and provided to be inserted into the conveying device (4) of the extracorporeal blood treatment machine (1), as well as at least two pressure-monitoring lines (14, 16, 18) each of which branches off the blood supply line (8a) or the blood return line (8b) at a branch (20, 22, 24); wherein
the pressure-monitoring lines (14, 16, 18) can be connected to the pressure sensor connections (26, 28, 30) to transmit pressure from the branches (20, 22, 24) to the corresponding pressure sensor connection (26, 28, 30),
**characterized in that**
the pressure sensor connections (26, 28, 30) are spaced apart from one another and positioned on the extracorporeal blood treatment machine (1) so as to match the tube set (8), and the length of the pressure-monitoring lines (14, 16, 18) as well as the positioning of the branches (20, 22, 24) are adapted such that, when the tube set (8) is mounted in the predefined arrangement on the connection mask (6), each pressure-monitoring line (14, 16, 18), owing to its limited length and the predefined arrangement of the associated branch (20, 22, 24) on the blood treatment machine, can be connected exclusively to a respective one of the pressure sensor connections (26, 28, 30).

2. The system (1) according to claim 1, **characterized in that** the connection mask (6) includes an arterial pressure sensor connection (26), a venous pressure sensor connection (28) and a blood treatment machine inlet-side pressure sensor connection (30) located to be spaced apart from one another and distributed in triangular form on the connection mask (6).

3. The system (1) according to any one of the claims 1 or 2, **characterized in that** one of the pressure sensor connections (26) is arranged to be lower on the connection mask (6), when viewed in the vertical direction of the blood treatment machine (1), than the at least one other pressure sensor connection (28, 30), preferably by a distance larger than or equal to the length of the associated pressure-monitoring line (14).

4. The system (1) according to claim 3, **characterized in that** one of the pressure sensor connections (26, 28, 30), preferably the arterial pressure sensor connection (26), is arranged to be lower than the conveying device (4), when viewed in the vertical direction of the blood treatment machine (1).

5. The system (1) according to any one of the claims 1 to 4, **characterized in that** the pressure sensor connections (26, 28, 30) are configured as, preferably identical, connectors, preferably Luer lock connectors, arranged to be connected to connectors, preferably Luer lock connectors, provided on the pressure-monitoring lines (14, 16, 18).

6. The system according to claim 1, **characterized in that** the pump section (5) is configured in the blood supply line (8a) and the blood supply line (8a) has, upstream of the pump section (5) in the conveying direction, an arterial branch (20) from which an arterial pressure-monitoring line (14) branches off which can be connected to an arterial pressure sensor connection (26) of the blood treatment machine (1) and includes, between the pump section (5) and a connector piece (12a) for connecting the blood supply line (8a) to an inlet of the blood treatment machine (2), a blood treatment machine inlet-side branch (24), from which a blood treatment machine inlet-side pressure monitoring line (18) branches off, which can be connected to a blood treatment machine inlet-side pressure sensor connection (30) of the blood treatment machine (1).

7. The system according to claim 1 or 6, **characterized in that** the blood return line (8b) includes, between a connector piece (12b) for connection to an outlet of the blood treatment machine (2) and an air trap (13), a venous branch (22) from which a venous pressure-monitoring line (16) branches off which can be connected to a venous pressure sensor connection (28) of the blood treatment machine (1).

8. The system according to claim 1, **characterized in that** at least one of the branches (20, 22, 24), preferably all branches (20, 22, 24), is/are configured as a separate branching element comprising a pressure oscillating diaphragm (POD) fluidically sealing the branch.

9. The system according to claim 8, **characterized in that** the at least one branch (20, 22, 24) branches off at a predetermined angle, preferably an angle between 45° and 90°, relative to the associated tube section of the blood supply line (8a) or the blood return line (8b) so that, when the tube set (8) is mounted in the predefined arrangement on the connection mask (6), the branch (20, 22, 24) is oriented toward the associated pressure sensor connection (26, 28, 30) on the blood treatment machine (1).

10. The system according to any one of the claims 1 or 6 to 9, **characterized in that** the pressure-monitoring lines (14, 16, 18) include, at their end remote from the branch (20, 22, 24), preferably identical, connectors, preferably Luer lock connectors, designed to be connected to the pressure sensor connections (26, 28, 30).

## Revendications

1. Système composé d'une machine de traitement extracorporel du sang (1) et d'un ensemble de tuyaux (8) pouvant être appliqué de manière amovible ou interchangeable et dans un agencement prédéterminé au niveau de la machine de traitement du sang (1), dans lequel
la machine de traitement extracorporel du sang (1) contient un dispositif de traitement du sang (2), un dispositif de transport (4) pour transporter du sang à travers le dispositif de traitement du sang (2) et un masque de raccordement (6) sur lequel des raccords de capteur de pression (26, 28, 30) sont disposés ; dans lequel
l'ensemble de tuyaux (8) contient au moins une conduite d'alimentation en sang (8a) pour alimenter en sang le dispositif de traitement du sang (2), une conduite de retour de sang (8b) pour renvoyer du sang traité depuis le dispositif de traitement du sang (2), une section de pompe (5) qui est adaptée et prévue pour être insérée dans le dispositif de transport (4) de la machine de traitement extracorporel du sang (1), ainsi qu'au moins deux conduites de surveillance de pression (14, 16, 18) qui partent chacune au niveau d'un embranchement (20, 22, 24) de la conduite d'alimentation en sang (8a) ou de la conduite de retour du sang (8b) ; dans lequel
les conduites de surveillance de pression (14, 16, 18) peuvent être connectées aux raccords de capteur de pression (26, 28, 30) pour transmettre une pression depuis les embranchements (20, 22, 24) au raccord de capteur de pression correspondant (26, 28, 30),
**caractérisé en ce que**
les raccords de capteur de pression (26, 28, 30) sont espacés les uns des autres et leur positionnement sur la machine de traitement extracorporel du sang (1) est adapté à l'ensemble de tuyaux (8), et la longueur des conduites de surveillance de pression (14, 16, 18) ainsi que le positionnement des embranchements (20, 22, 24) sont adaptés de sorte que, lorsque l'ensemble de tuyaux (8) est monté dans l'agencement prédéterminé sur le masque de raccordement (6), chaque conduite de surveillance de pression (14, 16, 18) puisse être raccordée exclusivement à l'un des raccords de capteur de pression (26, 28, 30) en fonction de sa longueur limitée et de l'agencement prédéterminé de l'embranchement correspondant (20, 22, 24) sur la machine de traitement du sang.

2. Système selon la revendication 1, **caractérisé en ce que** le masque de raccordement (6) présente un raccord de capteur de pression artérielle (26), un raccord de capteur de pression veineuse (28) et un raccord de capteur de pression (30) côté entrée du dispositif de traitement du sang, qui sont disposés espacés les uns des autres et répartis en forme de triangle sur le masque de raccordement (6).

3. Système selon l'une quelconque des revendications 1 ou 2, **caractérisé en ce que** l'un des raccords de capteur de pression (26) est disposé sur le masque de raccordement (6) plus bas que le au moins un autre raccord de capteur de pression (28, 30) vu en direction verticale de la machine de traitement du sang (1), de préférence selon une distance supérieure ou égale à la longueur de la conduite de surveillance de pression (14) correspondante.

4. Système selon la revendication 3, **caractérisé en ce que** l'un des raccords de capteur de pression (26, 28, 30), de préférence le raccord de capteur de pression artérielle (26), est disposé plus bas que le dispositif de transport (4) vu en direction verticale de la machine de traitement du sang (1).

5. Système selon l'une quelconque des revendications 1 à 4, **caractérisé en ce que** les raccords de capteur de pression (26, 28, 30) sont configurés en tant que connecteurs, de préférence identiques, de préférence des connecteurs Luer-Lock, qui sont configurés pour être connectés à des connecteurs, de préférence des connecteurs Luer-Lock, prévus sur les conduites de surveillance de pression (14, 16, 18).

6. Système selon la revendication 1, **caractérisé en ce que** la section de pompe (5) est formée dans la conduite d'alimentation en sang (8a), et la conduite d'alimentation en sang (8a) présente, en amont de la section de pompe (5) dans la direction de transport, un embranchement artériel (20) duquel part une conduite de surveillance de pression artérielle (14) qui peut être raccordée à un raccord de capteur de pression artérielle (26) de la machine de traitement du sang (1) et présente, entre la section de pompe (5) et une pièce de raccordement (12a) pour le raccordement de la conduite d'alimentation en sang (8a) à une entrée du dispositif de traitement du sang (2), un embranchement (24) côté entrée du dispositif de traitement du sang duquel part une conduite de surveillance de pression (18) côté entrée du dispositif de traitement du sang, qui peut être raccordée à un raccord de capteur de pression (30) côté entrée du dispositif de traitement du sang de la machine de traitement du sang (1).

7. Système selon la revendication 1 ou 6, **caractérisé en ce que** la conduite de retour de sang (8b) présente, entre une pièce de raccordement (12b) pour être raccordée à une sortie du dispositif de traitement du sang (2) et un piège à air (13), un embranchement veineux (22) duquel part une conduite de surveillance de pression veineuse (16) qui peut être raccordée à un raccord de capteur de pression veineuse (28) de la machine de traitement du sang (1).

8. Système selon la revendication 1, **caractérisé en ce qu'**au moins un des embranchements (20, 22, 24), de préférence tous les embranchements (20, 22, 24), est conçu en tant qu'élément d'embranchement séparé avec une membrane de pression oscillante (POD) assurant l'étanchéité fluidique de l'embranchement.

9. Système selon la revendication 8, **caractérisé en ce que** le au moins un embranchement (20, 22, 24) part selon un angle prédéterminé, de préférence un angle entre 45° et 90°, par rapport à la section de tuyau correspondante de la conduite d'alimentation en sang (8a) ou de la conduite de retour de sang (8b) de sorte que, lorsque l'ensemble de tuyaux (8) est monté sur le masque de raccordement (6) dans l'agencement prédéterminé, l'embranchement (20, 22, 24) soit orienté vers le raccord de capteur de pression correspondant (26, 28, 30) sur la machine de traitement du sang (1).

10. Système selon l'une quelconque des revendications 1 ou 6 à 9, **caractérisé en ce que** les conduites de surveillance de pression (14, 16, 18) présentent, au niveau de leur extrémité opposée à l'embranchement (20, 22, 24), des raccords ou connecteurs, de préférence identiques, de préférence des connecteurs Luer-Lock, qui sont configurés pour être connectés aux raccords de capteur de pression (26, 28, 30).
